# EUROPEAN PATENT SPECIFICATION

(11) **EP 4 017 445 B1**
(45) Date of publication and mention of the grant of the patent: **26.02.2025**
(21) Application number: 20751309.4
(22) Date of filing: 30.07.2020
(51) Int. Cl.: A61F 13/0203, A61F 13/05

(54) **SYSTEM FOR WOUND DRESSING MOISTURE MANAGEMENT USING FORCED AIRFLOW**
SYSTEM FÜR FEUCHTIGKEITSMANAGEMENT VON WUNDVERBÄNDEN MIT ZWANGSLUFTSTROM
SYSTÈME DE GESTION D'HUMIDITÉ DE PANSEMENT À L'AIDE D'UN FLUX D'AIR FORCÉ

(30) Priority: 22.08.2019 US 201962890316 P
(43) Date of publication of application: 29.06.2022
(73) Proprietor: Solventum Intellectual Properties Company, Maplewood, MN 55144 (US)
(72) Inventor: LOCKE, Christopher Brian, San Antonio, Texas 78265 (US)
(74) Representative: Simmons & Simmons
(86) International application number: PCT/IB2020/057196
(87) International publication number: WO 2021/033052

(56) References cited:
- WO-A1-2006/048240
- WO-A1-2006/048240
- WO-A1-2009/066106
- WO-A1-2009/066106
- WO-A1-2012/141999
- WO-A1-2012/141999
- WO-A1-2019/089522
- WO-A1-2019/089522
- WO-A2-2019/002086
- WO-A2-2019/002086
- US-A1- 2013 012 902
- US-A1- 2013 012 902
- US-A1- 2018 214 315
- US-A1- 2018 353 663
- US-A1- 2018 353 663

## Description

### BACKGROUND

The present disclosure relates generally to the field of treating wounds (e.g., burns, lacerations, surgical incisions, sores, ulcers, damaged tissue, nerve damage, etc.) and more particularly to managing excess moisture during the treatment of a wound.

During the treatment of a wound, the wound and surrounding skin (periwound) may be isolated from the ambient environment by a wound dressing to stimulate quicker healing. The wound dressing is typically defined by a fluid absorbency capacity that remains unchanged over time. Thus, once the fluid capacity of the wound dressing has been reached, the wound dressing will be incapable of absorbing or holding any additional fluid. In such situations, the exposure of the wound dressing to any additional fluid may put the wound dressing at a risk of leaking, swelling, and becoming detached.

Additionally, excess moisture within the wound dressing may decrease the effectiveness of wound treatment and lead to patient discomfort. In particular, excess moisture within a wound dressing may act as a thermal insulator, which induces sweating and patient discomfort. Moreover, sustained exposure to moisture increases the risk of tissue maceration-particularly when the wound dressing is applied over large areas of peri wound. To avoid the problems associated with excess moisture, users are often required to increase the frequency with which wound dressings are changed during the treatment of the wound. Wound treatment systems for managing moisture during the treatment of a wound are known for example from WO2012/141999 A1.

It would therefore be desirable to provide a wound treatment system configured to accelerate the evaporation of fluid from a wound dressing.

### SUMMARY

The invention is defined by the appended independent claim.

A selection of optional features of the invention is set out in the dependent claims.

Insofar as the term example or embodiment is used in the following, or features are presented as being optional, this should be interpreted in such a way that the only protection sought is that of the invention claimed. Reference(s) to "embodiment(s)" or "example(s)" throughout the description which are not under the scope of the appended claims merely represent possible exemplary executions and are not part of the present invention.

### BRIEF DESCRIPTION OF THE DRAWINGS

FIG. 1A illustrates a perspective view of a wound treatment system, according to an exemplary embodiment.
FIG. 1B illustrates a cross-sectional view of the wound treatment system of FIG. 1A, taken along line 1B-1B, according to an exemplary embodiment.
FIG. 1C illustrates a cross-sectional view of the wound treatment system of FIG. 1A, taken along line 1C-1C, according to an exemplary embodiment.
FIG. 2 illustrates an exploded perspective view of the wound treatment system of FIG. 1B.
FIG. 3 illustrates a perspective view of a moisture management system spacer structure and film layer, according to an exemplary embodiment.
FIG. 4 illustrates a perspective view of a moisture management system spacer structure and film layer, according to an exemplary embodiment.
FIG. 5 illustrates a perspective view of a moisture management system spacer structure and film layer, according to an exemplary embodiment.
FIG. 6 illustrates a perspective view of a moisture management system spacer structure and film layer, according to an exemplary embodiment.
FIG. 7 illustrates an exploded perspective view of a moisture management system spacer structure and film layer, according to an exemplary embodiment.
FIG. 8 illustrates an exploded perspective view of a moisture management system spacer structure and film layer, according to an exemplary embodiment.
FIG. 9 illustrates a perspective view of a wound treatment system, according to an exemplary embodiment.
FIG. 10 is a schematic representation of the flow of air through the wound treatment system during operation in a positive pressure mode, according to an exemplary embodiment.
FIG. 11 is a schematic representation of the flow of air through the wound treatment system during operation in a negative pressure mode, according to an exemplary embodiment.
FIG. 12 is a schematic representation of the flow of air through the wound treatment system during operation in a positive pressure mode, according to an exemplary embodiment.
FIG. 13 is a schematic representation of the flow of air through the wound treatment system during operation in a negative pressure mode, according to an exemplary embodiment.

### DETAILED DESCRIPTION

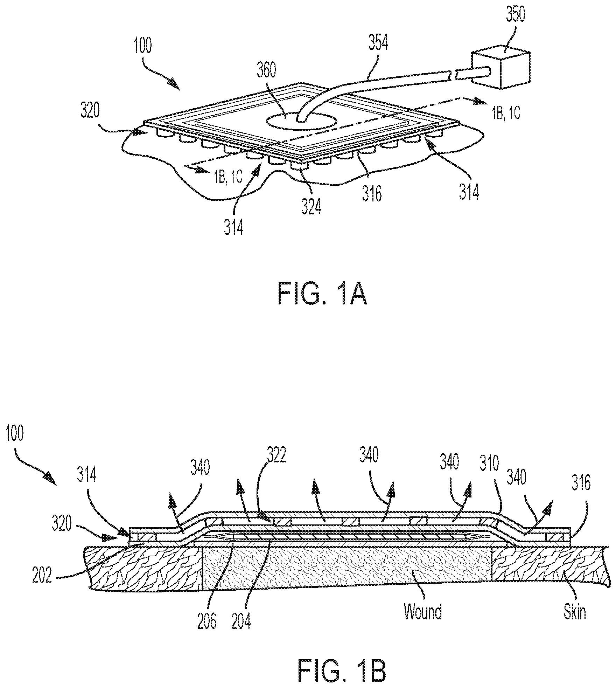

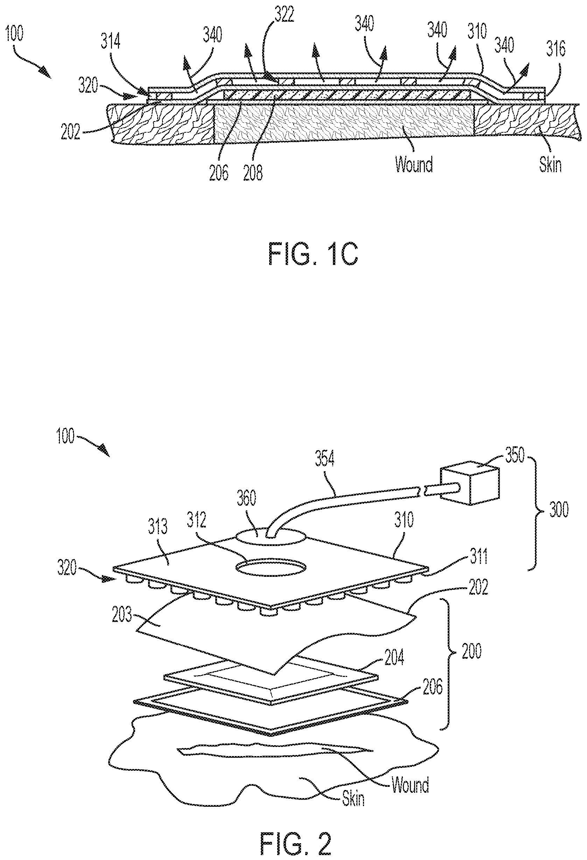

Referring to the FIGURES, a wound treatment system configured to facilitate the removal of excess moisture from a wound site is shown according to various exemplary embodiments. As illustrated in FIGS. 1A-1C and 2, the wound treatment system 100 generally includes a wound dressing 200 configured to be positioned around a wound (e.g. covering the wound), and a moisture management system 300 configured to increase the rate at which moisture is removed from a treatment space defined underneath the wound dressing 200.

Referring to FIGS. 1A-1C, and 2, the moisture management system 300 is configured to increase the rate at which moisture is removed from the wound dressing 200 by generating and directing a forced flow of dry air along an upper surface 203 of a liquid permeable drape layer 202 of the wound dressing 200. As the forced, dry air flows across the drape layer 202, moles of moisture which have permeated the drape layer 202 and pooled on an upper surface 203 thereof are lifted and removed by the dry air from the upper surface 203 of the drape layer 202. This removal of pooled moisture from the drape layer 202 allows additional moisture located within the treatment space to permeate through the liquid permeable drape layer 202. This accelerated diffusion and evaporation of liquid through the drape layer 202 resulting from the directed flow of dry air along the drape layer 202 generated by the moisture management system 300 increases the total fluid handling capacity of the wound dressing 200. Thus, the wound treatment system 100 actively extends the life of the static capacity of the wound dressing 200, which in turn prolongs the wear time of the wound dressing 200 without the need for an external reservoir to collect fluids removed from the treatment space. By reducing the frequency with which a wound dressing 200 needs to be changed, and by obviating the need for a patient to be tethered to an external fluid collection reservoir, the wound treatment system 100 advantageously minimizes the inconveniences to a user during the treatment of the wound. The ability of the wound treatment system 100 to accelerate the evaporation of fluid from within a wound dressing 200 may also advantageously further be used to minimize, or eliminate, patient discomfort typically associated with excess moisture at a wound site (such as, e.g., the increased risk of periwound maceration, increased sweating, etc.).

### I. Wound Dressing

The wound dressing 200 may be defined by a variety of different types of wound dressings. The wound dressing 200 may also be configured to treat a variety of different types of wounds, and for use in a variety of different types of wound treatments. The wound dressing 200 may be used as a stand-alone wound treatment, or may be a component of an additional wound therapy system with which the wound treatment system 100 is used, such as, e.g., a negative pressure wound treatment ("NPWT") system, an instillation therapy system, etc.

The wound dressing 200 includes a drape layer 202, and optionally any number and combination of additional layers. The number and selection of the additional dressing layers forming the wound dressing 200 may vary depending on a variety of factors, including, but not limited to: the type of wound being treated, the location of the wound being treated, the type of treatment being provided to the wound, the type of optional additional wound therapy system with which the wound treatment system 100 is used, etc. Non-limiting examples of additional dressing layers which may form a part of the wound dressing 200 include, e.g., an absorbent layer 204, an interface layer 206, a manifold layer 208, a wicking layer, etc.

### A. Drape Layer

The drape layer 202 of the wound dressing 200 supports the additional layers of the wound dressing 200 (e.g., the absorbent layer 204, the interface layer 206, the manifold layer 208, etc.) at the wound site. As described in more detail below, an upper surface 203 of the drape layer 202 delimits a lower portion of the flow path 340 of the wound treatment system 100.

As shown in FIGS. 1B and 1C, in some embodiments, a perimeter of the drape layer 202 extends beyond (e.g., circumscribes) a perimeter of the absorbent layer 204 (e.g., such as shown in FIG. 1B), a perimeter of the manifold layer 208 (e.g., as shown in FIG. 1C) and/or the perimeter of any other additional dressing layer(s) to provide an adhesive-coated margin for adhering the additional layers of the wound dressing 200 to the skin of a patient adjacent to the wound site being treated. In other embodiments, the adhesive-coated margin can be eliminated and wound dressing 200 can be adhered to the skin of a patient using other techniques.

The drape layer 202 may be formed from any number of different types of materials that are substantially impermeable to liquid and substantially permeable to moisture vapor. In other words, the drape layer 202 is permeable to water vapor, but not permeable to liquid water or wound exudate. This increases the total fluid handling capacity of the wound dressing 200 while promoting a moist wound environment. In some embodiments, the drape layer 202 is also impermeable to bacteria and other microorganisms. A suitable material for the drape layer 202 is a high moisture vapor transmission rate ("MVTR") material. As described in detail below, the wound treatment system 100 exploits the high MVTR of the drape layer 202 to manage fluids within the wound dressing 200. In some embodiments, the drape layer 202 is a thin layer of polyurethane film. One example of a suitable material for the drape layer 202 is the polyurethane film known as ESTANE 5714F. Other suitable polymers for forming the drape layer 202 include poly alkoxyalkyl acrylates and methacrylates, such as those described in Great Britain Patent Application No. 128063 1A filed November 22, 2002, the entire disclosure of which is incorporated by reference herein.

### B. Absorbent Layer

As illustrated by the wound treatment system 100 embodiment of FIGS. 1B and 2, the wound dressing 200 according to some embodiments is an absorbent wound dressing comprising an absorbent layer 204 that is adapted to collect (e.g. store) fluid from the wound site. The size and shape of the absorbent layer 204 may be varied as desired. As illustrated in FIGS. 1B and 2, in some embodiments the absorbent layer 204 comprises superabsorbent particles held within a liquid-tight envelope (e.g. a woven or non-woven pouch). Other non-limiting examples of absorbent layer 204 materials include superabsorbent fibers, superabsorbent polymers, hydrofibers, sodium carboxymethyl cellulose, alginates, sodium polyacrylate, hydrogels, hydrocolloids, etc.

### C. Manifold Layer

As illustrated by the wound treatment system 100 embodiment of FIG. 1C, the wound dressing 200 in various embodiments includes a manifold layer 208 configured to allow fluid (e.g. exudates, liquids, a vacuum created by the operation of the pump of the NPWT system) to be transmitted (e.g. distributed) to/from the wound. When treating highly exuding wounds, the manifold layer 208 may optionally include an open-cell foam marketed as GRANUFOAM^{™} by ACELITY^{™}. In other embodiments, it may be advantageous to retain some degree of fluid in the wound dressing 200 such that the wound does not entirely dry out (which may be harmful for healing). Accordingly, in such embodiments the manifold layer 208 may instead include a foam that is more hydrophilic than standard GRANUFOAM^{™}. In such embodiments, the ability of the manifold layer 208 to retain fluid may additionally assist in the fluid management of the wound dressing 200, with exudates and other fluids absorbed by the manifold layer 208 being evaporated by the forced airflow through the flow path 340 generated during operation of the air displacement device 350. Non-limiting examples of additional materials from which the manifold layer 208 may be formed include cellular foam, un-reticulated open-cell foam, porous tissue collections, gauze, felted mat, and/or any other material comprising a plurality of flow channels or pathways via which fluids may be distributed.

### D.Interface Layer

In various embodiments, the interface layer 206 is configured to reduce potential adherence of the wound dressing 200 to the wound and/or to prevent ingrowth of skin to the wound dressing 200. The interface layer 206 may be defined by a nonporous material comprising a plurality of perforations (fenestrations, holes, airways, windows, slits, etc.) extending therethrough that allow air and fluid to pass between the wound and the other layers of the wound dressing 200. In some embodiments, the interface layer 206 is made of a hydrophobic material such as polyethylene (PE) or other hydrophobic polymers.

According to some embodiments, the interface layer 206 may comprise a liquid-impermeable, elastomeric film having a plurality of bi-directional, pressure-responsive perforations formed therethrough. The interface layer 206 is optionally dimensioned such that an outer perimeter of the interface layer 206 is coextensive with, or slightly greater than the manifold layer 208 and/or absorbent layer 204 (e.g., the area of the interface layer 206 is between approximately 5% and 10% greater than the area of the manifold layer 208 and/or absorbent layer 204). The fluid restrictions define elastic passages that can expand (e.g. open) in response to a pressure gradient to allow flow therethrough. In the absence of a pressure gradient, the passages may be sufficiently small to form a seal or fluid restriction, thereby reducing or preventing liquid flow through the interface layer 206. Non-limiting examples of various materials from which the film defining the interface layer 206 in such embodiments may be formed include polyurethane, polyethylene, acrylics, polyolefin (such as cyclic olefin copolymers), polyacetates, polyamides, polyesters, copolyesters, PEBAX block copolymers, thermoplastic elastomers, thermoplastic vulcanizates, polyethers, polyvinyl alcohols, polypropylene, polymethylpentene, polycarbonate, styreneics, silicones, fluoropolymers, acetates, etc. In some such embodiments, the film may have thickness between approximately 50 microns and approximately 250 microns, and more specifically between approximately 100 microns and approximately 200 microns.

In some embodiments, the perforations comprise linear slots having a length less than approximately 4 millimeters (e.g. 3 millimeters), with adjacent slots spaced approximately 3 millimeters from one another. In other embodiments, the perforations may be defined by other configurations (e.g. holes) that are arranged in generally linear clusters having a length less than approximately 4 millimeters or less (e.g. 3 millimeters), with adjacent clusters optionally spaced approximately 3 millimeters from one another. In yet other embodiments, the perforations may be defined by any other variety of arrangements and dimensions.

### II. Moisture Management System

The moisture management system 300 operates to control moisture levels in a treatment space defined underneath the wound dressing 200 of the wound treatment system 100 by forcing airflow along an upper, outer surface of the wound dressing 200. Although the management of moisture levels in the treatment space may, in some embodiments, optionally be supplemented via the direct removal of fluids from the treatment space using a source of forced negative airflow (e.g. a pump) fluidly coupled to the treatment space-the management of moisture levels using the moisture management system 300 of the wound treatment system 100 described herein does not itself require fluid communication with the treatment space beneath the wound dressing 200. Rather, the forced airflow along the wound dressing 200 generated during operation of the moisture management system 300 indirectly removes moisture from the treatment space (i.e. removes moisture from the treatment space even in the absence of any fluid communication with the treatment space) by accelerating the diffusion and evaporation of fluid within the treatment space through the high MVTR drape layer 202 of the wound dressing 200.

As shown in FIGS. 1A-1C and 2, the moisture management system 300 generally includes a film layer 310, a spacer structure 320, and an air displacement device 350. The film layer 310 and spacer structure 320 are configured to be coupled (e.g. attached, supported by, adhered, etc.) to the wound dressing 200 to define a flow path 340 that extends along an upper (e.g., exterior, outer, etc.) surface of the wound dressing 200 (i.e., along the upper surface 203 of the drape layer 202). The air displacement device 350 is configured to be fluidly coupled to the flow path 340, with operation of the air displacement device 350 being used to generate air flow across the upper surface 203 of the drape layer 202 of the wound dressing 200.

Some or all of the components of the moisture management system 300 may be single use, or may optionally be configured to be re-used with different wound dressings 200. In some embodiments, the moisture management system 300 is configured for stand-alone treatment of a wound (i.e., wherein the wound treatment system 100 operates independent of any other wound therapy system or device). In other embodiments, the moisture management system 300 may be configured for use alongside other wound therapy systems, such as, e.g., an NPWT system.

### A. Film Layer

Referring to FIGS. 1A-1C, upon assembly of the wound treatment system 100, the film layer 310 (e.g. support layer) of the moisture management system 300 delimits an upper portion of a flow path 340 that extends along the upper surface 203 of the drape layer 202. As shown in FIG. 2, an opening 312 extending through the film layer 310 fluidly couples the flow path 340 to the air displacement device 350. When the wound treatment system 100 is operated in a positive pressure mode (described below), the opening 312 defines an inlet via which airflow generated by the air displacement device 350 enters the flow path 340. During operation of the wound treatment system 100 in a negative pressure mode (described below), the opening 312 defines an outlet via which air is evacuated from the flow path 340 by the air displacement device 350. As discussed below, the film layer 310 optionally also includes and/or defines a plurality of vents 314 via which air flows into or out from the flow path 340 during operation of the air displacement device 350.

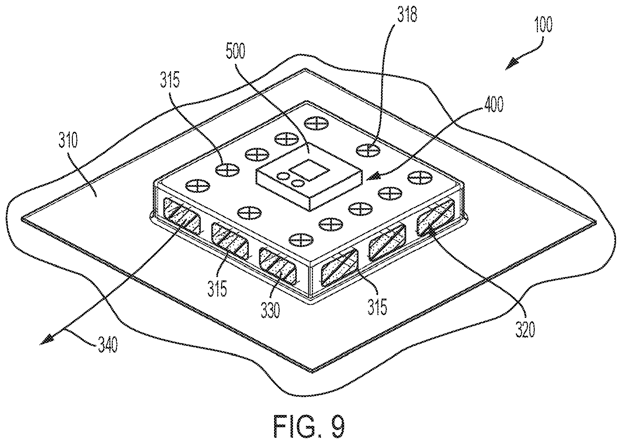

The film layer 310 optionally supports a connector 360 (e.g., a port, a SENSAT.R.A.C.^{™} connection pad marketed by ACELITY^{™}, etc.) via which the air displacement device 350 is coupled to the flow path 340. The connector 360 may indirectly couple the air displacement device 350 to the flow path 340 via a conduit 354 that is attached to and extends between the connector 360 and a remotely located air displacement device 350. Alternatively, in some embodiments, the air displacement device 350 and the connector 360 are components of an integrated module 400 (described in more detail below) that is supported relative to the film layer 310 (see, e.g., FIG. 9) around (e.g. adjacent to, surrounding, etc.) an outer periphery of the opening 312 in the film layer 310, such that the wound treatment system 100 defines a single, unitary structure.

In some embodiments, the opening 312 extends through a substantially central location of the film layer 310, so as to facilitate a generally uniform distribution of air flow along the upper surface of the wound dressing 200. In other embodiments, the fluid opening 312 (and corresponding connection between the air displacement device 350 and the flow path 340) may be arranged at other locations relative to the film layer 310 and/or wound dressing 200. For example, when treating a wound that exudes fluids at varying rates at different locations, the opening 312 is optionally formed at a location along the film layer 310 that will overlie a region of the wound that exudes fluid at a high rate. As another example, in embodiments in which the wound dressing 200 is attached at a location on a patient where gravity will result in the pooling of exudate at a lower portion of the wound dressing 200 (e.g. when the wound dressing 200 is attached to a leg, torso, upper arm, etc.), the fluid opening 312 may be formed at a location adjacent to a lower perimeter of the film layer 310.

The dimensions of the film layer 310 may be smaller, the same as, or larger than the dimensions of the wound dressing 200 of the wound treatment system 100. The dimensions of the film layer 310 relative to the wound dressing 200 may be selected based on an intended treatment protocol. For example, given the detrimental effect moisture may have on a periwound, in various embodiments the film layer 310 is sized to be coextensive with, or extend beyond, the outer perimeter of the wound dressing 200 to which the moisture management system 300 is attached, such as, e.g., representatively illustrated in FIGS 1A-1C. Such an arrangement advantageously allows the flow path 340 to be defined across the entirety of the upper surface 203 of the drape layer 202-including those portions of the drape layer 202 that overlay the periwound. In other embodiments, the film layer 310 is dimensioned smaller than the wound dressing 200, such that a flow path 340 is defined along only a designated portion of the upper surface 203 of the drape layer 202. Such a configuration may be advantageous in situations in which it is desired to reduce levels of moisture at a specific location along a wound without significantly effecting moisture levels at other locations along the wound (such as, e.g. may occur during the treatment of larger wounds) and/or to minimize unnecessary power consumption resulting from the operation of the air displacement device 350 to cause air flow across a greater area than needed.

In some embodiments, the film layer 310 is attached to an underlying surface (e.g. an upper surface of the wound dressing 200, the skin of a patient, etc.) in situ during the attachment of the wound treatment system 100 to the patient. In such embodiments, adhesive is optionally provided along portions, or the entirety, of the perimeter of the lower surface 311 of the film layer 310 to facilitate the attachment of the film layer 310 to the underlying surface. Alternatively, the film layer 310, spacer structure 320 and drape layer 202 are optionally provided as a single, integrated assembly, in which the perimeter of the film layer 310 is preattached (via, e.g., adhesive, welding, etc.) to the drape layer 202.

### B. Vents

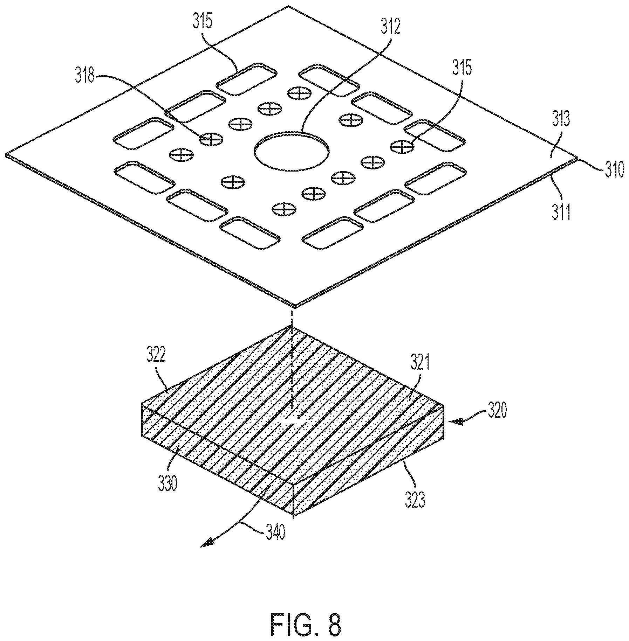

As noted above, the film layer 310 includes and/or defines one or more vents 314 via which air is forced out from or into the flow path 340 during operation of the air displacement device 350. In some embodiments, such as, e.g., shown in FIGS. 8 and 9, the vents 314 include openings 315 (e.g. apertures, etc.) that extend through the film layer 310.

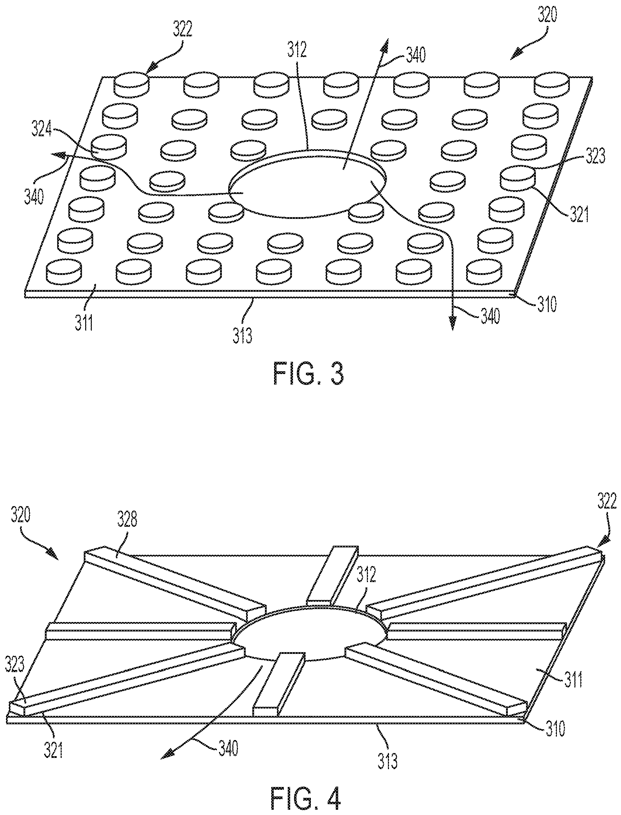

As shown in FIGS. 1A-1C, the vents 314 may also optionally be defined by unattached portions 316 of a perimeter of the film layer 310 that are not directly coupled to an underlying surface (e.g. the wound dressing 200, the skin of a patient, etc.). As illustrated by FIGS. 1B, 1C and 3, in some such embodiments the flow elements 322 of the spacer structure 320 (described below) that are located around (e.g. adjacent to, surrounding, etc.) the perimeter of the film layer 310 are formed having a greater height (e.g., the flow elements 322 extend further downward relative to an upper surface 313 of the film layer 310) than flow elements 322 that are located along an interior portion of the lower surface 311 of the film layer 310. Such an increased height of the flow elements 322 located around the outer perimeter of the film layer 310 is configured to ensure that the unattached portions 316 of the outer periphery of the film layer 310 remain offset from the underlying surface to which the film layer 310 is attached (e.g. the wound dressing 200, the skin of the patient, etc.) and do not occlude flow into/out from the flow path 340.

In embodiments in which the wound treatment system 100 is configured to be operated in a negative pressure mode, valves and/or filters are optionally located within some or all of the vents 314. During operation of the wound treatment system 100 in the negative pressure mode, the optional valves (e.g., slit valves 318) are configured to provide control over the rate of ambient air flow into the flow path 340, while the optional filters are configured to prevent microorganisms or other harmful particles from entering the flow path 340 (and air displacement device 350).

### C. Spacer Structure

The spacer structure 320 (e.g. spacer assembly, support structure) of the moisture management system 300 vertically offsets (e.g., spaces apart, separates, elevates, etc.) the lower surface 311 of the film layer 310 from the upper surface 203 of the drape layer 202 upon assembly of the wound treatment system 100. The space (e.g., void, air gap, etc.) defined between the lower surface 311 of the film layer 310 and the upper surface 203 of the drape layer 202 as a result of this vertical offset defines a portion of the flow path 340 of the wound treatment system 100.

The spacer structure 320 includes one or more flow elements 322 each having an outer surface that extends between a first end 321 (e.g., an upper end) and a second end 323 (e.g., a lower end). Upon assembly of the wound treatment system 100, the spacer structure 320 is arranged relative to the wound dressing 200 and film layer 310 so that the first ends 321 of the flow elements 322 are located adjacent and extend downwards relative to the lower surface 311 of the film layer 310. The second ends 323 of the flow elements 322 are located adjacent to and extend upwards relative to the upper surface 203 of the drape layer 202.

To facilitate assembly of the wound treatment system 100, the second ends 323 of the flow elements 322 are optionally coated with an adhesive via which the spacer structure 320 can be attached relative to the upper surface 203 of the drape layer 202. In embodiments in which the spacer structure 320 and film layer 310 are not monolithically, or otherwise integrally formed, the upper ends 321 of the spacer structure 320 may also optionally be coated with an adhesive via which the spacer structure 320 can be attached relative to the lower surface 311 of the film layer 310. In yet other embodiments, the drape layer 202, spacer structure 320 and film layer 310 are provided a single, integrated structure, such that assembly of the wound treatment system 100 in situ requires no more effort than would typically be required to assemble a wound dressing comprising a separately attachable backing layer (e.g. a drape layer).

As illustrated by the embodiments of FIGS. 3-8, the flow elements 322 may be defined by a variety of different structures having any number of different shapes, sizes and configurations. According to various embodiments, the flow element(s) 322 of the spacer structure 320 are provided as separate and discrete structure(s) from the film layer 310, and are arranged relative to the film layer 310 and wound dressing 200 during assembly of the wound treatment system 100.

To facilitate the assembly of the wound treatment system 100, in other embodiments the spacer structure 320 and film layer 310 are optionally provided as a single, integral one-piece structure. In some such embodiments, the spacer structure 320 is monolithically formed with the film layer 310, with the flow elements 322 including structures that are defined by the lower surface 311 of the film layer 310. In other such embodiments, the flow elements 322 defining the spacer structure 320 include discrete structures that are attached to the lower surface 311 of the film layer 310.

Illustrated in FIGS. 3 and 4 are exemplary embodiments of integral, one-piece film layer 310 and spacer structure 320 assemblies. As shown in FIG. 3, the flow elements 322 in some such embodiments include adhesive dots 324 (e.g. beads, islands, patches, etc.) formed from a cured semi-permanent or permanent adhesive (e.g., viscose UV curing adhesive). The adhesive dots 324 define discrete structures that are adhered at their respective first ends 321 to locations along the lower surface 311 of the film layer 310. Upon assembly of the wound treatment system 100, the second ends 323 of the adhesive dots 324 adhere the film layer 310 relative to the upper surface 203 of the drape layer 202. The adhesive dots 324 are formed having a thickness (i.e. height), such that the first ends 321 are laterally offset from the second ends 323 by a distance that allows the adhesive dots 324 to support the film layer 310 in a spaced and separated arrangement relative to the wound dressing 200 upon assembly of the wound treatment system 100. Although the adhesive dots 324 are shown as being provided pre-adhered to the film layer 310 to define an integral spacer structure 320/film layer 310 assembly, an adhesive composition may alternatively be applied to the lower surface 311 of the film layer 310 (and/or to the upper surface 203 of the drape layer 202) to form the adhesive dots 324 along the lower surface 311 of the film layer 310 and/or along the upper surface 203 of the drape layer 202 during assembly of the wound treatment system 100.

As illustrated by the embodiment of FIG. 4, instead of adhesive dots 324, the discrete and separate flow elements 322 that define a single, unitary spacer structure 320 and film layer 310 assembly may instead include one or more flow walls 328 that are attached (e.g. adhered) to the lower surface 311 of the film layer 310. Notably, in such embodiments, the flow wall(s) 328 can be defined by a variety of different shapes, sizes, and configurations.

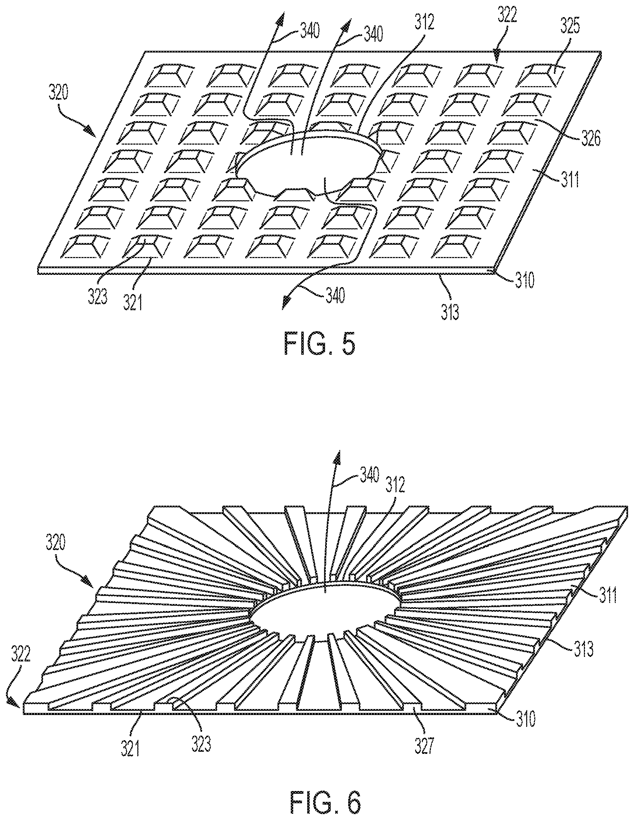

Exemplary embodiments of spacer structures 320 that are monolithically formed with the film layer 310 are shown in FIG. 5 and 6. As shown in FIG. 5, in some such embodiments, the lower surface 311 of the film layer 310 is textured, and includes a plurality of peaks 325 that are separated from one another by valleys 326. The flow elements 322 correspond to the peaks 325 of the textured surface, and are defined by portions of the lower surface 311 of the film layer 310 that extend further downward from an upper surface 313 of the film layer 310 (i.e. have an increased thickness) as compared to the portions of the lower surface 311 of the film layer 310 defining the valleys 326. Although the peaks 325 defining the flow elements 322 are shown as being truncated pyramids, the peaks 325 may be formed into a variety of other different shapes (e.g. ridges, ribs, posts, etc.).

Turning to FIG. 6, in other embodiments, a lower surface 311 of the film layer 310 is embossed to define a plurality of raised portions 327 (i.e. non-embossed portions of the film layer 310). In such embodiments of a spacer structure 320 formed monolithically with the film layer 310, the flow elements 322 are defined by the raised portions 327 of the lower surface 311. Alternatively (or additionally), the upper surface 313 of the film layer 310 may be debossed, and the flow elements 322 are defined by the downwardly projecting, debossed portions of the upper surface 313 of the film layer 310 (not shown).

According to some embodiments, instead of the spacer structure 320 and film layer 310 being provided as an integral, one-piece assembly, the spacer structure 320 and drape layer 202 may alternatively be provided as an integral, one-piece assembly.

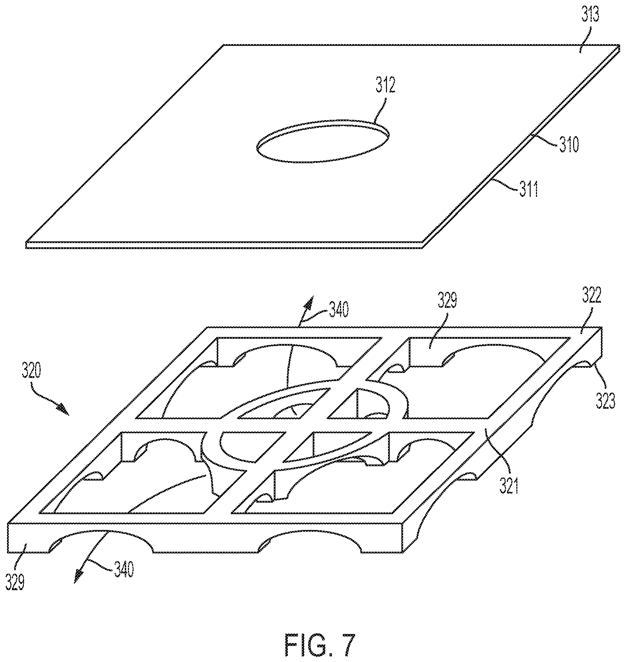

Shown in FIGS. 7 and 8 are exemplary embodiments of spacer structures 320 that are provided separately and discretely from the film layer 310, and which are arranged and optionally attached relative to the film layer 310 during assembly of the wound treatment system 100. As shown in FIG. 7, in some such embodiments the flow elements 322 include a plurality of struts 329 (e.g. walls) that are interconnected to form a scaffold which defines the spacer structure 320.

Referring to FIG. 8, in other embodiments, the discretely and separately provided spacer structure 320 is defined by a porous layer 330. The porous layer 330 may be formed of a variety of different porous materials. For example, the porous layer 330 optionally includes a felted foam (having, e.g., a thickness of between approximately 1cm and 3cm), or a non-felted foam (having, e.g., a thickness of less than approximately 5mm). In such embodiments, the porous material from which the porous layer 330 is formed defines the flow element 322 of the spacer structure 320. According to some embodiments, the porous material (e.g. felted or non-felted foam) used to form the porous layer 330 that defines the space structure 320 of FIG. 8 may also be used to form the flow elements 322 of other spacer structure 320 embodiments, such as, e.g., flow walls 328 (see, e.g., FIG. 4), struts 329 (see, e.g., FIG. 7), etc.

As also illustrated by FIGS. 3-8, the shape, size, and arrangement of flow elements 322 of the spacer structure 320 may be varied as desired, such as, e.g., to define a flow path 340 that directs air in a manner along specific portions of the upper surface of the wound dressing 200. For example, in embodiments in which it is desired to manage moisture across the entirety of the wound dressing 200, the flow elements 322 may be arranged such that flow channels defined between adjacent flow elements 322 are fluidly interconnected and define a single flow path 340 that extends across the entirety of the portion of the upper surface 203 of the drape layer 202 (e.g., as representatively illustrated by the arrangement of flow elements 322 in FIGS. 3, 5 7 and 8).

Alternatively, it may instead be desired to direct dry air along only specific target locations along the wound dressing 200, such as, e.g., to portions of the flow path 340 that overlie the periwound, or which overlie highly exuding regions of the wound. Accordingly, in some embodiments, the shape, size, and arrangement of flow elements 322 may be selected to define one or more enclosed flow channels that are discrete and fluidly isolated from other portions of the flow path 340 (such as, e.g., representatively illustrated by the embodiments of FIGS. 4 and 6).

In yet other embodiments, the arrangement of flow elements 322 in a non-uniform distribution, and/or the selection of flow elements 322 having varying sizes may be used to accelerate the rate of moisture removal at the target locations. For example, the arrangement of flow elements 322 in a greater density and/or the arrangement of flow elements 322 having larger dimensioned second ends 323 at locations relative to portions of the wound dressing 200 that do not correspond to target locations minimizes the degree to which air flowing through the flow path 340 passes along the upper surface 203 of the drape layer 202 at such non-target locations. Resulting, less moisture is acquired by the air flowing through the flow path 340 prior to the air reaching a target location (e.g. a portion of the flow path 340 overlaying the periwound). Because drier air has a greater capacity to lift moisture from the upper surface 203 of the drape layer 202, the decreased moisture level of the air flowing through the flow path 340 along the target locations increases the rate at which moisture may be removed from the target locations. In other embodiments, a similar effect of delivering drier air flow to desired target locations may also be accomplished using an optional blocking structure (e.g. a thicker layer of adhesive, an intermediate layer of material, etc.) that is arranged between the spacer structure 320 and drape layer 202 along the portions of the wound dressing 200 that do not correspond to the target locations.

### D. Air Displacement Device

The air displacement device 350 may include any number of different air-moving devices (e.g., manual or automatic pump, blower, bellows, etc.). As shown in FIG. 1, upon assembly of the wound treatment system 100, a remotely located air displacement device 350 is fluidly coupled to the flow path 340 via a conduit 354. Alternatively (as described in detail below), the air displacement device 350 is provided as a component of a module 400 that is supported by the film layer 310, such that the assembled wound treatment system 100 defines a single, self-contained device (see, e.g., FIG. 9).

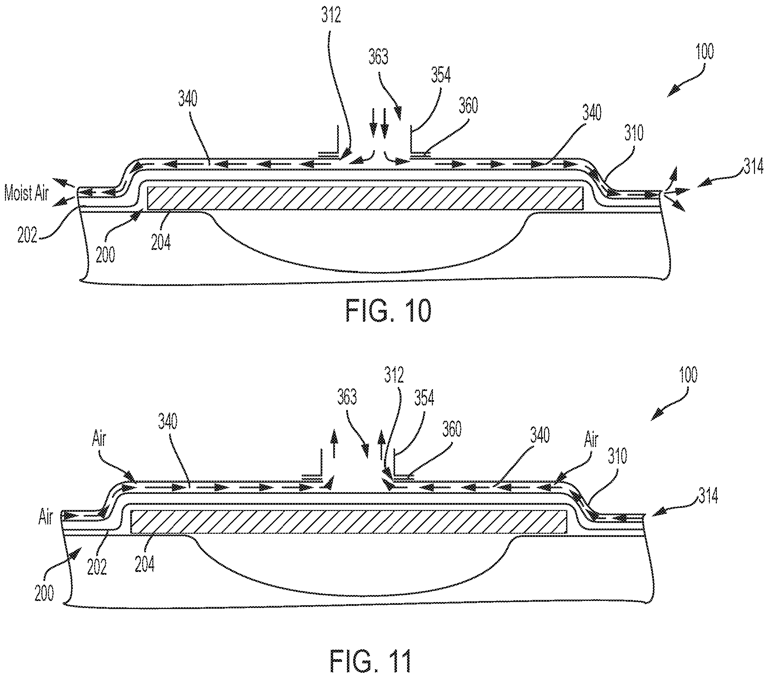

Operation of the air displacement device 350 is configured to generate a flow of air through the flow path 340 of the wound treatment system 100. As illustrated in FIG. 10, operation of the air displacement device 350 in a positive pressure mode causes air to flow from the air displacement device 350, through the opening 312 in the film layer 3 10, through the flow path 340 defined by the flow elements 322 of the spacer structure 320, through one or more vents 314, and into the ambient environment. As shown in FIG. 11, operation of the air displacement device 350 in a negative pressure mode generates a vacuum that evacuates air through the opening 312 in the film layer 310 that has been drawn into the flow path 340 via the vents 314. During some operations of the wound treatment system 100, the air displacement device 350 forces air into (or out from) the flow path 340 at a rate of between approximately 0.5L/min and approximately 2L/min, which creates a backpressure of approximately 10mmHg.

In embodiments in which the wound treatment system 100 is used alongside an additional wound therapy system incorporating a pump or other air moving device, the air displacement device 350 may be provided in addition to the pump of the additional wound treatment system (e.g., a NPWT system), such that such that the wound treatment system 100 is capable of being operated independently and separately from the NPWT system (or other wound treatment system).

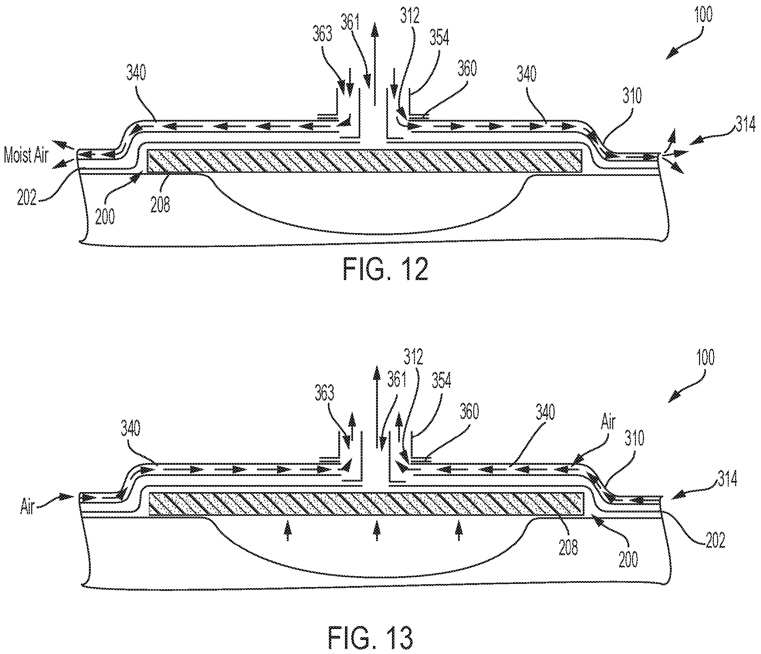

Alternatively, in other embodiments, the pump of the NPWT system (or other additional wound therapy system with which the wound treatment system 100 is used) defines the air displacement device 350. As illustrated in FIGS. 13 and 14, in some such embodiments the shared pump is fluidly coupled to a connector 360 comprising a NPWT vacuum conduit 361 fluidly coupled to a space defined underneath the drape layer 202, and a moisture management conduit 363 fluidly coupled to the flow path 340. The connector 360 optionally also includes a pressure monitoring conduit that is fluidly coupled to one or both of the treatment space underneath the drape layer 202 and the flow path 340.

The shared pump that defines each of the NPWT system (or other additional wound therapy system with which the wound treatment system 100 is used) and the air displacement device 350 may alternate between being operated to provide treatment using the NPWT system and providing treatment using the wound treatment system 100. In such embodiments, the connector 360 optionally includes one or more valves via which the shared pump is selectively fluidly coupled to either the NPWT conduit 361 or the moisture management conduit 363 based on the desired treatment. For example, the connector 360 includes an optional proportional leak valve configured to fluidly couple the pump to moisture management conduit 363 while fluidly isolating the NPWT conduit 361 during operation of the shared pump to generate pressures in excess of a predetermined threshold (e.g. at negative pressures above 150mmHg). During operation of the shared pump at pressures below the predetermined threshold, the valve is configured to fluidly couple the pump to the NPWT conduit 361, while fluidly isolating the moisture management conduit 363.

During other operations of the wound treatment system 100 and NPWT system (or other additional wound therapy system with which the wound treatment system 100 is used), the shared pump that defines the air displacement device 350 is operated to simultaneously provide treatment using each treatment system. As illustrated by FIG. 12, in some such embodiments, the wound treatment system 100 is operated in a negative pressure mode concurrently with the operation of the pump to provide NPWT treatment. In such embodiments, an optional valve of the connector 360 is selectively actuated such that each of the NPWT conduit 361 and moisture management conduit 363 are fluidly coupled to the pump.

As shown in FIG. 13, the wound treatment system 100 can also be operated in the positive pressure mode concurrently with the operation of the pump to provide NPWT treatment. In such embodiments, an outlet of the pump is coupled (e.g. via a valve) to the moisture management conduit 363, with that exhaust airflow resulting from the operation of the pump to generate a vacuum for the NPWT treatment being used as the positive forced airflow that is delivered to the flow path 340 via the moisture management conduit 363. In other embodiments, an optional valve is selectively actuated to restrict (e.g. reduce the diameter of) a portion of the NPWT conduit 361 located upstream of a Venturi tube (or a selectively openable passageway) that fluidly couples the NPWT conduit 361 and moisture management conduit 363. The decrease in pressure resulting from air flow through the restriction in the NPWT conduit 361 causes positive forced airflow to be directed through the Venturi tube (or passageway) and into the flow path 340.

### E. Module

As noted above, and representatively illustrated by the wound treatment system 100 embodiment of FIG. 9, the wound treatment system 100 may optionally be formed as an integral self-contained system that may be supported entirely by a patient. In such embodiments, the air displacement device 350, as well as any additional optional components of the wound treatment system 100 (e.g. a controller 500, sensors, a power source, a charging circuit, a communications interface, etc.) are housed in an optional module 400. The module 400 may optionally also support a patient interface comprising a display and/or user inputs (e.g., buttons) via which information may be provided to and/or received from a user during operation of the wound treatment system 100. In embodiments in which the module 400 is configured to operate the wound treatment system 100 in the negative pressure mode and/or in embodiments in which the wound treatment system 100 is used in conjunction with a NPWT system, the module 400 may also contain a reservoir (e.g., an absorbent material, a canister, etc.) configured to store fluid that has been evaporated from the wound dressing 200 and/or that is evacuated from the treatment space defined underneath the wound dressing 200 and/or any other number of components of the additional wound therapy system.

The module 400 optionally includes an engagement structure configured allow the module 400 to be sealingly secured around (e.g. adjacent to, surrounding, etc.) an outer periphery of the opening 312 in the film layer 310 via an engagement to an optional connector 360 supported relative to the film layer 310. In other embodiments, the module 400 may also be secured directly to the film layer 310 using adhesive, welding, etc. In embodiments in which the module 400 is intended to be reused with additional wound dressings 200, bacterial and/or hydrophobic filters 380 are optionally provided between the opening 312 in the film layer 310 and an inlet of the module 400. For example, filters 380 may be attached to any one or more of the opening 312 in the film layer 310, the connector 360, and the engagement structure of the module 400 to prevent fluid and/or bacterial ingress and contamination of the module 400 during use of the wound treatment system 100 and/or during attachment or removal of the module 400 to or from wound dressing 200.

### III. Controller

Various operations of the wound treatment system 100 may be controlled by an optional controller 500 (shown, e.g., in FIG. 9). For example, the controller 500 may turn the air displacement device 350 on/off, and/or can be used to vary the speed at which the air displacement device 350 is operated. The controller 500 may also provide selective control over the actuation of any optional valves of the connector 360 during operation of the wound treatment system 100 (e.g. to switch operation of the air displacement device 350 between positive and negative pressure modes, to switch between the type of treatment being provided during operation of the wound treatment system 100 in conjunction with an additional wound therapy system, etc.).

The controller 500 may be configured to operate the various components of the wound treatment system 100 (e.g. the air displacement device 350, valves, etc.) based on a variety of different inputs received from any number of different sources. For example, in some embodiments, the controller 500 may operate the wound treatment system 100 based on inputs received from a user. In other embodiments, the controller 500 may be configured to operate the wound treatment system 100 in accordance to one or more preprogrammed modes stored in a memory of the controller 500. For example, the controller 500 may operate the wound treatment system 100 in an alternating pressure mode, in which operation of the air displacement device 350 is periodically varied between operation in the positive pressure mode, and operation in the negative pressure mode. Such periodic alternation between positive and negative air flow may advantageously be used disrupt regions of dense flow within the treatment space underneath the wound dressing 200 and to allow moisture to be removed more uniformly from the wound.

In yet other embodiments, the controller 500 is configured to operate the wound treatment system 100 responsive to readings or measurement obtained from one or more sensors (e.g. moisture sensor, humidity sensor, pressure sensor, etc.) incorporated into the wound treatment system 100. According to an exemplary embodiment, the wound treatment system 100 includes a moisture level sensor assembly comprising one or more moisture indicators and an optical reader configured to detect changes in the indicators. Given that the fluid capacity of and/or fluid levels within the wound dressing 200 may vary over time, the indicators are advantageously selected to be capable of providing a dynamic, two-way (i.e. reversible), near real-time indication as to both increases and decreases in moisture levels within the wound dressing 200 over the course of use of the wound treatment system 100.

One non-limiting example of such a reversible, dynamic indicator that may be used in the wound treatment system 100 is described in more detail in co-pending application US 62/784,187, filed December 21, 2018 and titled "FLUID STATUS INDICATOR FOR A WOUND DRESSING," the entirety of which is incorporated herein by reference. In addition to providing a dynamic representation of moisture levels in the treatment space underneath the wound dressing 200, the indicators described in the above referenced co-pending application also advantageously are positionable entirely underneath the wound dressing 200 (i.e. do not extend through the drape layer 202). Such an incorporation of indicators into the wound treatment system 100 allows the wound dressing 200 to remain integral and sealed, and thus advantageously minimizes the risk of exudates, or other fluids or substances from the treatment space underneath the wound dressing 200, contaminating the components of the optionally included module 400.

The moisture indicators are incorporated into (i.e. positioned in direct contact with) one or both of the drape layer 202 and an additional dressing layer (e.g., an absorbent layer 204, a manifold layer 208, etc.), so as to be capable of dynamically responding to changes in moisture levels within the wound dressing 200 (e.g. changes in the fluid capacity of the absorbent layer 204, moisture levels at a portions of the drape layer 202 overlaying the periwound, etc.). The indicators are advantageously positioned directly below the drape layer 202 to facilitate the ability of the optical reader to detect changes in the visual appearance of the indicators through the drape layer 202. A fusible fiber (or other attachment mechanism) optionally secures the indicators relative to the wound dressing 200 to ensure contact of the indicators with the absorbent layer 204 and/or to ensure that the indicators are aligned relative to the module 400 such that visual transformations of the indicators are capable of being detected by the optical reader. The optical reader is optionally incorporated into the module 400, or is otherwise positionable relative to the upper surface of the wound treatment system 100.

During some uses of the wound treatment system 100, the controller 500 is optionally configured to operate the air displacement device 350 in a manner configured to minimize power consumption. For example, during the operation of the wound treatment system 100 to prevent moisture levels from exceeding a static fluid capacity of a wound dressing 200, the controller 500 may optionally be configured to initiate operation of the air displacement 350 to generate airflow through the flow path 340 only in response to the detection of a visual transition of an indicator indicative of the moisture level in the treatment space underneath the wound dressing 200 being equal to, or exceeding, a predetermined upper threshold moisture level. Additionally, or alternatively, in some embodiments the controller 500 may optionally be configured to initiate operation of the air displacement 350 to generate airflow through the flow path 340 in response to the detection of a visual transition of an indicator indicative of the moisture level at the periwound being equal to, or exceeding, a predetermined upper threshold moisture level.

Upon detecting (e.g. via a visual transition of the indicators) that the moisture level has decreased to a level equal to or below an intermediate moisture level, the controller 500 is optionally configured to reduce the speed at which the air displacement device 350 is operated. Once the moisture level within the treatment space has reached a level equal to or below a predetermined lower threshold (e.g. between approximately 40% and less than approximately 50% of the fluid capacity of the wound dressing 200), the controller 500 ceases operation of the air displacement device 350 to save power until such a time when the moisture level in the treatment space (e.g. a moisture level of an optional absorbent layer 204, a moisture level at the periwound, etc.) is detected to again be equal to or greater than the upper predetermined threshold. Such selective operation of the air displacement device 350 may continue one or more times during the operation of the wound treatment system 100.

In some embodiments, the controller 500 may be configured to continuously receive readings and measurements from one or more sensors (e.g., from a moisture level sensor assembly). Alternatively-to further conserve power-the controller 500 may instead be configured to selectively obtain sensor readings only at predetermined intervals (e.g., every 3-5 minutes). In some such embodiments, the predetermined interval at which the sensor readings are obtained may vary in response to the detection of various threshold sensor readings. For example, the controller 500 may be configured to obtain moisture level readings at a more frequent interval following the detection (using, e.g., the moisture level sensor assembly) that a moisture level underneath the wound dressing 200 is equal to, or exceeds, an upper moisture level threshold.

In various embodiments, the air displacement device 350 optionally comprises a plurality of independently operable air displacement devices 350 that are fluidly coupled at various locations to the flow path 340 around (e.g. adjacent to, surrounding, etc.) the outer peripheries of various openings 312 in the film layer 310. In such embodiments, the operation of some or all of the air displacement devices 350 is optionally independently controllable, allowing moisture levels to be managed as needed at specific target areas along the wound dressing 200. In some embodiments, each individual air displacement device 350 is individually controllable responsive to a moisture level reading (or other sensed condition) obtained from a moisture indicator (or other sensor) positioned at a location relative to the wound that corresponds to the location at which the respective air displacement device 350 is positioned. In addition to allowing airflow to be directed to specific desired target locations, such selective activation of only those air displacement devices 350 that are needed to provide the desired delivery of airflow also further assists in decreasing the power consumption of the wound treatment system 100.

### IV. Configuration of Exemplary Embodiments

The construction and arrangement of the systems and methods as shown in the various exemplary embodiments are illustrative only. Although only a few embodiments have been described in detail in this disclosure, many modifications are possible (e.g., variations in sizes, dimensions, structures, shapes and proportions of the various elements, values of parameters, mounting arrangements, use of materials, colors, orientations, etc.). For example, the position of elements can be reversed or otherwise varied and the nature or number of discrete elements or positions can be altered or varied. The order or sequence of any process or method steps can be varied or re-sequenced according to alternative embodiments. Other substitutions, modifications, changes, and omissions can be made in the design, operating conditions and arrangement of the exemplary embodiments.

As utilized herein, the terms "approximately," "about," "substantially", and similar terms are intended to have a broad meaning in harmony with the common and accepted usage by those of ordinary skill in the art to which the subject matter of this disclosure pertains. It should be understood by those of skill in the art who review this disclosure that these terms are intended to allow a description of certain features described and claimed without restricting the scope of these features to the precise numerical ranges provided.

## Claims

1. A wound treatment system (100), comprising:
a wound dressing (200) comprising a drape layer (202), and a moisture management system (300) comprising:
a film layer (310);
a spacer assembly comprising:
one or more flow elements (322) each having an outer surface which extends between a first end (321) and a second end (323), the first ends being located adjacent to and extend downwards relative to a lower surface (311) of the film layer (310), and the second ends (323) being located adjacent to and extending upwards relative to the upper surface (203) of the drape layer (202);
the spacer assembly defining a space between the lower surface (311) of the film layer (310) and the upper surface (203) of the drape layer (202) defining a flow path (340) between the upper and lower surfaces (203, 311) and the one or more flow elements (322)
and
an air displacement device (350) sealingly engaged with an opening (312) formed in and extending through the film layer (310) and configured to cause air to flow through the flow path (340);
wherein the spacer assembly and film layer (310) define a monolithic structure;
wherein the lower surface (311) of the film layer (310) is embossed to define the one or more flow elements (322) as raised portions (327) of the lower surface (311), and/or the upper surface (313) of the film layer (310) is debossed to define the one or more flow elements (322) as downwardly projecting, debossed portions of the upper surface (313) of the film layer (310).

2. The wound treatment system (100) of claim 1, wherein the air displacement device (350) is entirely supported atop an upper surface of the film layer (310).

3. The wound treatment system (100) of claim 1, wherein the air displacement device (350) comprises a connector structure sealingly attached around the opening in the film layer (310), the connector structure being fluidly connected to a pump located remotely from the film layer (310) by a conduit.

4. The wound treatment system (100) of claim 1, wherein the wound dressing (200) further includes an absorbent layer configured to store fluid exuded from a wound.

5. The wound treatment system (100) of claim 1, wherein the absorbent layer (204) comprises a superabsorbent material.

6. The wound treatment system (100) of claim 5, wherein the superabsorbent material is contained within a pouch formed from a non-woven material.

7. The wound treatment system (100) of claim 1, wherein the wound dressing (200) further includes a manifold layer (208) configured to allow fluid to be transmitted to the wound.

8. The wound treatment system (100) of claim 7, wherein the manifold layer (208) comprises an open-cell foam.

9. The wound treatment system (100) of claim 1, wherein the wound dressing (200) further includes a non-porous wound interface layer (206).

10. The wound treatment system (100) of claim 9, wherein the wound interface (206) comprises a polyurethane film.

11. The wound treatment system (100) of claim 9, wherein the film includes a plurality of perforations extending therethrough.

## Patentansprüche

1. Ein Wundbehandlungssystem (100), aufweisend:
einen Wundverband (200), aufweisend eine Abdeckschicht (202), und ein Feuchtigkeitsmanagementsystem (300), aufweisend:
eine Folienschicht (310);
eine Abstandshalteranordnung, aufweisend:
ein oder mehrere Strömungselemente (322), die jeweils eine Außenoberfläche aufweisen, die sich zwischen einem ersten Ende (321) und einem zweiten Ende (323) erstreckt, wobei sich die ersten Enden benachbart zu einer Unterseite (311) der Folienschicht (310) befinden und sich relativ zu dieser nach unten erstrecken, und sich die zweiten Enden (323) benachbart zu der Oberseite (203) der Abdeckschicht (202) befinden und sich relativ zu dieser nach oben erstrecken;
wobei die Abstandshalteranordnung einen Raum zwischen der unteren Oberfläche (311) der Folienschicht (310) und der oberen Oberfläche (203) der Abdeckschicht (202) definiert und einen Strömungsweg (340) zwischen der oberen und unteren Oberfläche (203, 311) und dem einen oder den mehreren Strömungselementen (322) definiert.
und
eine Luftverdrängungsvorrichtung (350), die mit einer Öffnung (312) abdichtend in Eingriff ist, die in der Folienschicht (310) ausgebildet ist und sich durch diese erstreckt und konfiguriert ist, um zu bewirken, dass Luft durch den Strömungsweg (340) strömt;
wobei die Abstandshalteranordnung und die Folienschicht (310) eine monolithische Struktur definieren;
wobei die untere Oberfläche (311) der Folienschicht (310) geprägt ist, um das eine oder die mehreren Strömungselemente (322) als erhöhte Abschnitte (327) der unteren Oberfläche (311) zu definieren, und/oder die obere Oberfläche (313) der Folienschicht (310) blindgeprägt ist, um das eine oder die mehreren Strömungselemente (322) als nach unten ragende, blindgeprägte Abschnitte der oberen Oberfläche (313) der Folienschicht (310) zu definieren.

2. Das Wundbehandlungssystem (100) nach Anspruch 1, wobei die Luftverdrängungsvorrichtung (350) auf einer oberen Oberfläche der Folienschicht (310) vollständig getragen wird.

3. Das Wundbehandlungssystem (100) nach Anspruch 1, wobei die Luftverdrängungsvorrichtung (350) eine Verbindungsstruktur aufweist, die um die Öffnung in der Folienschicht (310) herum abdichtend angebracht ist, wobei die Verbindungsstruktur mit einer Pumpe, die sich entfernt von der Folienschicht (310) befindet, durch eine Leitung fluidisch verbunden ist.

4. Das Wundbehandlungssystem (100) nach Anspruch 1, wobei der Wundverband (200) ferner eine absorbierende Schicht einschließt, die konfiguriert ist, um Fluid aufzubewahren, das aus einer Wunde abgesondert wird.

5. Das Wundbehandlungssystem (100) nach Anspruch 1, wobei die absorbierende Schicht (204) ein superabsorbierendes Material aufweist.

6. Das Wundbehandlungssystem (100) nach Anspruch 5, wobei das superabsorbierende Material innerhalb eines Beutel aus Vliesmaterial enthalten ist.

7. Das Wundbehandlungssystem (100) nach Anspruch 1, wobei der Wundverband (200) ferner eine Verteilerschicht (208) einschließt, die konfiguriert ist, um zu ermöglichen, dass Fluid an die Wunde übertragen wird.

8. Das Wundbehandlungssystem (100) nach Anspruch 7, wobei die Verteilerschicht (208) einen offenzelligen Schaumstoff aufweist.

9. Das Wundbehandlungssystem (100) nach Anspruch 1, wobei der Wundverband (200) ferner eine nicht-poröse Wundgrenzflächenschicht (206) einschließt.

10. Das Wundbehandlungssystem (100) nach Anspruch 9, wobei die Wundgrenzfläche (206) einen Polyurethanfilm aufweist.

11. Das Wundbehandlungssystem (100) nach Anspruch 9, wobei die Folie eine Vielzahl von Perforationen, die sich durch sie hindurch erstrecken, aufweist.

## Revendications

1. Système de traitement de plaie (100), comprenant :
un pansement pour plaie (200) comprenant une couche de champ (202) et un système de gestion d'humidité (300) comprenant :
une couche de film (310) ;
un ensemble d'espacement comprenant :
un ou plusieurs éléments d'écoulement (322) ayant chacun une surface externe qui s'étend entre une première extrémité (321) et une seconde extrémité (323), les premières extrémités étant localisées adjacentes à, et s'étendent vers le bas par rapport à, une surface inférieure (311) de la couche de film (310), et les secondes extrémités (323) étant localisées adjacentes à, et s'étendant vers le haut par rapport à, la surface supérieure (203) de la couche de champ (202) ;
l'ensemble d'espacement définissant un espace entre la surface inférieure (311) de la couche de film (310) et la surface supérieure (203) de la couche de champ (202) définissant un trajet d'écoulement (340) entre les surfaces supérieure et inférieure (203, 311) et le ou les éléments d'écoulement (322)
et
un dispositif de déplacement d'air (350) en prise étanche avec une ouverture (312) formée dans, et s'étendant à travers, la couche de film (310) et configuré pour amener de l'air à s'écouler à travers le trajet d'écoulement (340) ;
dans lequel l'ensemble d'espacement et la couche de film (310) définissent une structure monolithique ;
dans lequel la surface inférieure (311) de la couche de film (310) est en relief pour définir le ou les éléments d'écoulement (322) en guise de parties surélevées (327) de la surface inférieure (311), et/ou la surface supérieure (313) de la couche de film (310) est en creux pour définir le ou les éléments d'écoulement (322) en guise de parties en creux, faisant saillie vers le bas, de la surface supérieure (313) de la couche de film (310).

2. Système de traitement de plaie (100) selon la revendication 1, dans lequel le dispositif de déplacement d'air (350) est entièrement supporté au-dessus d'une surface supérieure de la couche de film (310).

3. Système de traitement de plaie (100) selon la revendication 1, dans lequel le dispositif de déplacement d'air (350) comprend une structure de raccord attachée de manière étanche autour de l'ouverture dans la couche de film (310), la structure de raccord étant raccordée fluidiquement à une pompe localisée à distance de la couche de film (310) par un conduit.

4. Système de traitement de plaie (100) selon la revendication 1, dans lequel le pansement pour plaie (200) comporte en outre une couche absorbante conçue pour stocker un fluide exsudant d'une plaie.

5. Système de traitement de plaie (100) selon la revendication 1, dans lequel la couche absorbante (204) comprend un matériau superabsorbant.

6. Système de traitement de plaie (100) selon la revendication 5, dans lequel le matériau superabsorbant est contenu au sein d'une poche formée d'un matériau non tissé.

7. Système de traitement de plaie (100) selon la revendication 1, dans lequel le pansement pour plaie (200) comporte en outre une couche de collecteur (208) conçue pour permettre à un fluide d'être transmis à la plaie.

8. Système de traitement de plaie (100) selon la revendication 7, dans lequel la couche de collecteur (208) comprend une mousse à alvéoles ouvertes.

9. Système de traitement de plaie (100) selon la revendication 1, dans lequel le pansement pour plaie (200) comporte en outre une couche non poreuse d'interface de plaie (206).

10. Système de traitement de plaie (100) selon la revendication 9, dans lequel l'interface de plaie (206) comprend un film de polyuréthane.

11. Système de traitement de plaie (100) selon la revendication 9, dans lequel le film comporte une pluralité de perforations s'étendant à travers celui-ci.
